# EUROPEAN PATENT APPLICATION

(11) **EP 1 164 171 A2**
(43) Date of publication of application: **19.12.2001**
(21) Application number: 01305029.9
(22) Date of filing: 08.06.2001
(51) Int. Cl.: C08L 83/07

(54) **Silicone compositions**

(30) Priority: 12.06.2000 US 592362
(71) Applicant: GENERAL ELECTRIC COMPANY, Schenectady, NY 12345 (US)
(72) Inventor: Kilgour, John Alfred, Clifton Park, New York 12065 (US); Chaiyawat, Atchara, Ballston Lake, New York 12019 (US); Nye, Susan Adams, Feura Bush, New York 12067 (US)
(74) Representative: Szary, Anne Catherine, Dr.

(57) **Abstract**

A silicone material containing a polymer network formed by polymerization of a polyfunctional organosilicone compound, wherein the polyfunctional organosilicone compound contains alkenyl radicals and hydride radicals on the same molecule, is useful as a component in personal care compositions

## Description

The invention relates to silicone compositions, more particularly to compositions comprising a silicone polymer network.

The personal care industry thrives on being able to deliver multiple performance products based on mixture of several components, with each having performance characteristics vital to the final formulation. One critical characteristic is the ability to provide a silky initial feel derived from low molecular weight silicones in the formulation while maintaining a high, but shear-thinnable, viscosity. While these low molecular weight silicones provide the desired feel characteristics, they are also low viscosity, highly flowable liquids. Thus they are not easily held in a formulation, preferring rather to separate and flow out of a given container or flow uncontrollably when used in a specific application. Further, it is desirable to achieve this while providing a smooth, low-residue feel on dry-down. US Patent Nos. 5,493,041 and 4,987,169 and coassigned US Patent No. 5,760,116 each disclose the use of polymeric silicone materials prepared in volatile silicone oils to deliver the desirable initial feel of volatile, low viscosity silicones to formulations while at the same time provide high viscosity and a smooth silky feel on dry-down.

Each of the disclosed silicone elastomers is derived from the combination of a chain extending organosiloxane and a crosslinking agent along with a hydrosilylation catalyst and silicone oil. The crosslinking agent is a minor component to the final polymer formulation. Since the ratio of functional groups on the chain extending organosiloxane to the functional groups on the crosslinking agent strongly influence the performance properties of the silicone elastomer, it is a disadvantage, particularly in commercial production, to require very precise measurements of very small amounts of the crosslinking agent for addition to large amounts of the chain extending organosiloxane. Further, the reaction requires complete mixing of the gelling agent and the functional organopolysiloxane. If the reaction starts before complete mixing occurs, or if the gelling agent congregates in domains, then a different, lower performing gel network may be formed.

In a first aspect, the present invention relates to a silicone material, comprising:
(a) a polymer network, said polymer network comprising a polymerized product of a polyfunctional organosilicone compound, said polyfunctional organosilicone compound comprising, on average per molecule of the compound, one or more alkenyl substituents and one or more silicon-bonded hydride substituents, and
(b) a fluid within the network.

In a second aspect, the present invention relates to a method for making a silicone material, comprising polymerizing a polyfunctional organosiloxane compound, said polyfunctional organosiloxane compound comprising, on average per molecule of the compound, one or more alkenyl radicals and one or more silicon-bonded hydride radicals, in the presence of a fluid to form a polymer network with the fluid within the network.

In a third aspect, the present invention is directed to a method for making a polyfunctional organosilicone compound, comprising equilibrating one or more linear or cyclic siloxanes with a silylhydride functional siloxane and an alkenyl functional siloxane in the presence of a linear phosphonitrile chloride equilibration catalyst

In a fourth aspect, the present invention relates to a personal care composition, comprising a polymerized product of a polyfunctional organosilicone compound, said polyfunctional organosilicone compound comprising, on average per molecule of the compound, one or more alkenyl substituents and one or more silicon-bonded hydride substituents.

In a fifth aspect, the present invention comprises a method for making a personal care composition, comprising combining one or more personal care ingredients with a polymerized product of a polyfunctional organosilicone compound, said polyfunctional organosilicone compound comprising, on average per molecule of the compound, one or more alkenyl substituents and one or more silicon-bonded hydride substituents.

In a sixth aspect, the present invention relates to a method for reversibly imparting characteristics of a solid to a fluid, comprising combining the fluid with a polymer network, said network comprising a polymerized product of a polyfunctional organosilicone compound, said polyfunctional organosilicone compound comprising, on average per molecule of the compound, one or more alkenyl substituents and one or more silicon-bonded hydride substituents and said network being swellable by the fluid, so that the fluid is contained within the polymer network.

The polymerized product of the polyfunctional organosilicone compound exhibits a high affinity for emollient fluids. Personal care compositions containing the polymerized product and an emollient fluid, whether the polymerized product and an emollient fluid are separately added or added in the form of the silicone material of the present invention, exhibit improved sensory feel and exhibit good stability, that is, a high resistance to phase separation.

In a preferred embodiment, the silicone material of the present invention comprises, based on 100 parts by weight ("pbw") of the silicone material, from 0.1 pbw to 99 pbw, more preferably from 1.0 pbw to 90 pbw, even more preferably from 2 pbw to 40 pbw, of the polymer network and from 1 pbw to 99.9 pbw, more preferably from 10 pbw to 99 pbw, even more preferably from 60 pbw to 98 pbw, of the fluid.

As used herein, the terminology "polymer network" means a three dimensionally extending structure made up of polymer chains. In a preferred embodiment, the polymer network is a cross-linked structure wherein the polymer chains of the network are interconnected, preferably via covalent chemical bonds. Preferably, in those embodiments of the present invention which comprise a polymer network and a fluid within the network, the fluid component is contained within interstices of the polymer network. As used herein, the term "interstices" is used in reference to the polymer network to denote spaces within the polymer network, that is, spaces between the polymer chains of the polymer network.

The polyfunctional organosilicone compound of the composition of the present invention comprises both alkenyl and silyl hydride substituents on the same molecule, such as, for example, the organopolysiloxanes disclosed in coassigned U.S. Patent Nos. 5,698,654 and 5,753,751, the disclosure of which is hereby incorporated herein by reference.

In a preferred embodiment, the polyfunctional organosilicone compound comprises:
one or more first structural units of the formula (I):

R¹ ₐSiO_{4-a/2} (I)

wherein each R¹ is independently a monovalent hydrocarbon radical, provided that at least one R¹ group per unit is an alkenyl radical, and a is an integer wherein 0 ≤ a ≤ 3, and
one or more second structural units of the formula (II):

R² _{b}SiO_{4-b/2} (II)

wherein each R² is independently H or a monovalent hydrocarbon radical, provided that at least one R² group per unit is H, and b is an integer wherein 0 ≤ b ≤ 3.

As used herein "monovalent hydrocarbon radical" includes monovalent acyclic hydrocarbon radicals, monovalent alicyclic hydrocarbon radicals and monovalent aromatic hydrocarbon radicals.

As used herein, the terminology "acyclic hydrocarbon radical" means a monovalent straight chain or branched hydrocarbon radical, preferably containing from 1 to 20 carbon atoms per radical, which may be saturated or unsaturated and which may be optionally substituted or interrupted with one or more functional groups, such as, for example, carboxyl, cyano, hydroxy, halo and oxy. Suitable monovalent acyclic hydrocarbon radicals include, for example, alkyl, alkenyl, alkynyl, hydroxyalkyl, cyanoalkyl, carboxyalkyl, carboxamide, alkylamido and haloalkyl, such as, for example, methyl, ethyl, sec-butyl, tert-butyl, octyl, decyl, dodecyl, cetyl, stearyl, ethenyl, propenyl, butynyl, hydroxypropyl, cyanoethyl, carboxymethyl, chloromethyl and 3,3,3-fluoropropyl.

As used herein the term "alkyl" means a saturated straight or branched monovalent hydrocarbon radical. In a preferred embodiment, monovalent alkyl groups are selected from linear or branched alkyl groups containing from 1 to 12 carbons per group, such as, for example, methyl, ethyl, propyl, iso-propyl, n-butyl, iso-butyl, sec-butyl, tert-butyl, pentyl, hexyl, heptyl, decyl, dodecyl.

As used herein the term "alkenyl" means a straight or branched monovalent terminally unsaturated hydrocarbon radical, preferably containing from 2 to 10 carbon atoms per radical, such as, for example, ethenyl, 2-propenyl, 3-butenyl, 5-hexenyl, 7-octenyl and ethenylphenyl.

As used herein, the terminology "monovalent alicyclic hydrocarbon radical" means a monovalent radical containing one or more saturated hydrocarbon rings, preferably containing from 4 to 10 carbon atoms per ring, per radical which may optionally be substituted on one or more of the rings with one or more alkyl radicals, each preferably containing from 2 to 6 carbon atoms per group, halo radicals or other functional groups and which, in the case of a monovalent alicyclic hydrocarbon radical containing two or more rings, may be fused rings. Suitable monovalent alicyclic hydrocarbon radicals include, for example, cyclohexyl and cyclooctyl.

As used herein, the terminology "monovalent aromatic hydrocarbon radical" means a monovalent hydrocarbon radical containing one or more aromatic rings per radical, which may, optionally, be substituted on the aromatic rings with one or more alkyl radicals, each preferably containing from 2 to 6 carbon atoms per group, halo radicals or other functional groups and which, in the case of a monovalent aromatic hydrocarbon radical containing two or more rings, may be fused rings. Suitable monovalent aromatic hydrocarbon radicals include, for example, phenyl, tolyl, 2,4,6-trimethylphenyl, 1,2-isopropylmethylphenyl, 1-pentalenyl, naphthyl, anthryl.

In a highly preferred embodiment, the polyfunctional organosilicone compound comprises one or more organopolysiloxanes of the formula (III):

M_{c}M^{vi} _{d}M^{H} ₑD_{f}D^{vi} _{g}D^{H} ₕTᵢT^{vi} ⱼT^{H} ₖQ₁ (III)

wherein:
M is R³₃SiO_{1/2},
M^{vi} is R⁴₂R⁵SiO_{1/2},
M^{H} is R⁶₂R⁷SiO_{1/2}
D is R⁸₂SiO_{2/2},
D^{vi} is R⁹R¹⁰SiO_{2/2},
D^{H} is R¹¹R¹²SiO_{2/2},
T is R¹³SiO_{3/2},
T^{vi} is R¹⁴SiO_{3/2},
T^{H} is R¹⁵SiO_{3/2},
Q is SiO_{4/2},
R³, R⁴, R⁶, R⁸, R⁹, R¹¹ and R¹³ are each monovalent non-alkenyl hydrocarbon radicals,
R⁵, R¹⁰ and R¹⁴ are each alkenyl,
R⁷, R¹² and R¹⁵ are each H, and
c, d, e, f, g, h, i, j, k and 1 are each integers selected to provide polymer a having a viscosity of from 1 to 1,000,000 cSt, more preferably from 1 to 100,000 cSt, and having a desired amount of alkenyl groups and silicon-bonded H radicals per molecule.

In a preferred embodiment, each R³, R⁴, R⁶, R⁸, R⁹, R¹¹ and R¹³ is independently alkyl, hydroxyalkyl, a polyhydric alcohol radical, monocyclic aromatic, aralkyl, oxaalkylene or alkylcarbonyloxaalkylene.

More preferably, each R³, R⁴, R⁶, R⁸, R⁹, R¹¹ and R¹³ is independently (C₁-C₆₀)alkyl, hydroxy(C₁-C₁₂)alkyl, a polyhydric alcohol radical according to formula (IV), (V) or (VI)

- R¹⁶-CHOHCH₂OH (IV)

- R¹⁷-CHOHCH₂CH₂OH (V)

- R¹⁸-C(R¹⁹)₃ (VI)

wherein each R¹⁶, R¹⁷ and R¹⁸ is independently (C₁-C₁₂)alkylene or (C₁-C₁₂)oxaalkylene and each R¹⁹ is independently H, hydroxy, (C₁-C₁₂)alkyl, or hydroxy(C₁-C₁₂)alkyl, provided that at least two R¹⁹ substituents per radical are hydroxy or hydroxy(C₁-C₁₂)alkyl,
aralkyl according to the formula (VII): 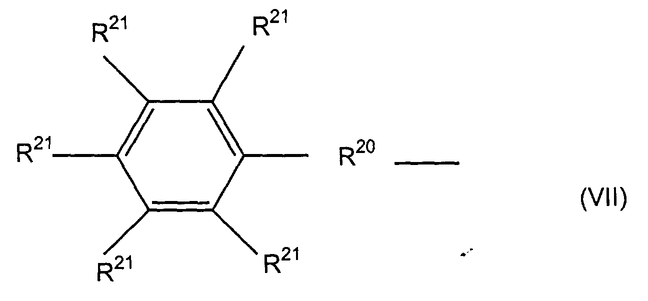 wherein R²⁰ is (C₁-C₆)alkylene and each R²¹ is independently H, hydroxyl, (C₁-C₆)alkyl, hydroxy(C₁-C₆)alkyl, or - OCOR²², wherein R²² is (C₁-C₆)alkyl,
oxaalkylene according to formula (VIII) or (IX):

-(CH₂)ₘO(CR²³H)ₙ - (VIII)

-(CH₂)ₒ(O(CR²⁴H)ₚ)_{q}(CH₂)ᵣ- (IX)

wherein each R²³ and R²⁴ is independently H or alkyl, preferably (C₁-C₈)alkyl, and each m, n, o, p, q and r is independently an integer of from 1 to 20, or alkylcarbonyloxaalkylene according to formula (X):

- R²⁵-C-R²⁶ ₃ (X)

wherein R²⁵ is (C₁-C₁₂)alkylene or (C₁-C₁₂)oxaalkylene and each R²⁶ is independently H, (C₁-C₂₄)alkyl, or - OCOR²⁷, wherein each R²⁷ is independently (C₁-C₂₄)alkyl, provided that at least one R²⁶ group per radical is - OCOR²⁷.

In a highly preferred embodiment, each R³, R⁴, R⁶, R⁸, R⁹, R¹¹ and R¹³ is independently (C₂₀-C₆₀)alkyl, 2-phenylethyl, 2-methyl-2-phenylethyl, a polyhydric alcohol radical according to formula (XI) or (XII) 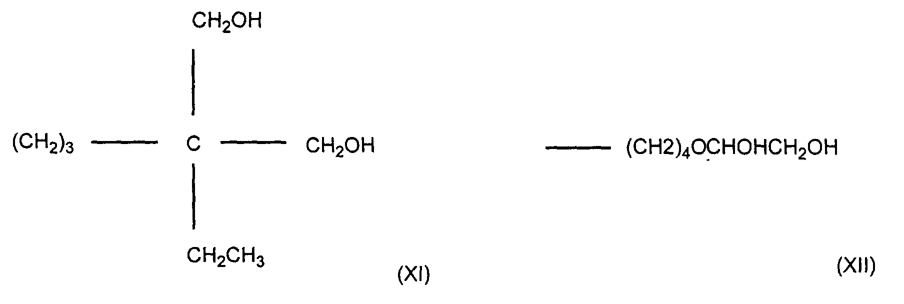 an oxaalkylene radical according to formula (XIII):

- CH₂O(CH₂CH₂O)ₛ(CH₂CH₂CH₂O)ₜH (XIII)

wherein s and t are each integers of from 0 to 50, provided that g and h cannot both be 0, or
an alkylcarbonyloxaalkylene according to formula (XIV): 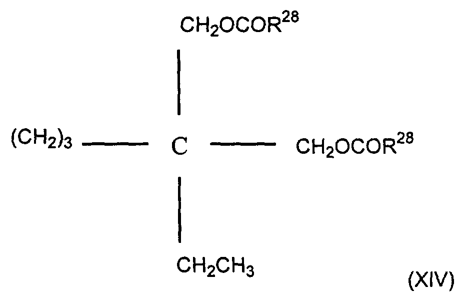 wherein each R²⁸ is independently (C₁-C₂₄)alkyl.

In a preferred embodiment, the polyfunctional organosilicone compound contains, on average, greater than 0, preferably greater than or equal to 0.5, alkenyl radicals per molecule and greater than 0, preferably greater than or equal to 0.5, silicon-bonded H groups per molecule.

In a first embodiment, the polyfunctional organosilicone compound contains, on average, from greater than 0 to less than 1.5 alkenyl radicals per molecule and from greater than 0 to less than 1.5 silicon-bonded H groups per molecule.

In a second embodiment, the polyfunctional organosilicone compound contains, on average, from greater than 0 to less than 1.5 alkenyl radicals per molecule and greater than or equal to 1.5 silicon-bonded H groups per molecule.

In a third embodiment, the polyfunctional organosilicone compound contains, on average, greater than or equal to 1.5 alkenyl radicals per molecule and from greater than 0 to less than 1.5 silicon-bonded H groups per molecule.

In a fourth embodiment, the polyfunctional organosilicone compound contains, on average, greater than or equal to 1.5, more preferably greater than or equal to 2, even more preferably from 2 to 10, alkenyl radicals per molecule and greater than or equal to 1.5, more preferably greater than or equal to 2, even more preferably from 2 to 50, silicon-bonded H groups per molecule.

In an alternative embodiment, the polyfunctional organosilicone compound contains, on average, from one to 1.5 alkenyl radical and from one to 1.5 silicon-bonded H group per molecule.

In a preferred embodiment, the polyfunctional organosilicone contains from 0.001 to 100, more preferably from 0.01 to 5 alkenyl radicals per 100 silicon atoms of the polyfunctional organosilicone compound and from 0.001 to 100, more preferably from 0.01 to 5 silicon-bonded hydrogen groups per 100 silicon atoms of the polyfunctional organosilicone compound.

In a preferred embodiment, the polyfunctional organosilicone is an organopolysiloxane of the structural formula (XV)

M^{Vi}DᵤD^{H} ᵥM^{Vi} (XV)

wherein M^{Vi}, D, D^{H} and M^{Vi} are each defined as above and u and v are each integers, wherein 0 ≤ u ≤ 1000, preferably 200 ≤ u ≤ 800, and 1 ≤ v ≤ 10, preferably 2 ≤ v ≤ 8.

Coassigned US Patent No. 5,753,751 to Liao and Nye describe one method of generating polyfunctional organosilicone compounds. In a preferred embodiment, the polyfunctional organosilicone compound is generated via an equilibration reaction of the desired functional components.

Alternatively, the polyfunctional organosilicone compound may be generated by equilibrating a linear, such as, for example, a silanol fluid, or a cyclic siloxane, such as, for example, D₄, with a silylhydride source, such as, for example MD^{H}₅₀M, wherein M and D^{H} are each described as above and an alkenyl functional siloxane source, such as, for example M^{vi}D₉₀₀M^{vi}, wherein M^{vi} and D are each described as above, in the presence of a suitable equilibration catalyst, such as, for example, a linear phosphonitrile chloride ("LPNC") catalyst.

The present invention also allows synthesis of branched polyfunctional organosilicone compounds through incorporation of T and Q units in the siloxane chain.

The polymer network of the present invention is formed, at least in part, by polymerization of the polyfunctional organosilicone compound in the presence of a hydrosilylation catalyst, such as, for example, platinum. Optionally, this may be done with added solvent that is compatible with the ingredients and does not interfere with the hydrosilylation reaction. Examples of suitable solvents include low molecular weight silicones, isopropyl alcohol, and toluene.

In a preferred embodiment, the polymer network of the present invention is formed by polymerizing the polyfunctional organosilicone compound in the presence of a hydrosilylation catalyst and at least a portion of the fluid component of the present invention.

The method of polymer synthesis provides for incorporation of a wide range of organofunctional groups into the copolymeric structure. Thus, the inclusion of other organofunctional groups, such as, for example, organic epoxides, epoxysiloxanes, terminally unsaturated organic and alkenylsiloxane compounds can be used to modify the resulting copolymers.

In one embodiment, the organofunctional groups are introduced to the network as R³, R⁴, R⁶, R⁸, R⁹, R¹¹ and R¹³ radicals present on an polyfunctional organosiloxane according to formula (III) above. In an alternative embodiment, the organofunctional groups are introduced to the network during polymerization of the polyfunctional organosiloxane by including organofunctional compounds to the reaction mixture which are copolymerizable with the polyfunctional organosiloxane under the chosen polymerization reaction conditions. For example, the polyfunctional organosilicone compound may be polymerized in the presence of other reactants, such as for example alkenyl functional silicone compounds, alkenyl functional organic compounds or silylhydride functional compounds which contain the desired organodfunctional groups and which areare reactive with or copolymerizable with the polyfunctional silicone compound under the conditions used to polymerize the polyfunctional silicone compound and accordingly, the polymer network may include structural units derived from such other reactants.

In the preparation of the silicone elastomers claimed in US Patent Nos. 5,760,116 and 4,987,169, substituents added during the reaction can only add via reaction with the silylhydride crosslinking agent. Thus their modifying effects are limited to small portions of the silicone elastomer and in some cases so localized that their effectiveness may be reduced. According to the present invention, the polyfunctional organosilicone compound may, optionally, be further substituted, either in prior to or during the polymerization to form the network, with other organic substituents. Unlike the previous silicone elastomers, the optional substituents may be evenly distributed in polymer network of the present invention.

Fluids suitable for use as the fluid component of the composition of the present invention are those compounds or mixtures of two or more compounds that are in the liquid state at or near room temperature, for example, from about 20°C about 50°C, and about one atmosphere pressure, and include, for example, silicone fluids, hydrocarbon fluids, esters, alcohols, fatty alcohols, glycols and organic oils. In a preferred embodiment, the fluid component of the composition of the present invention exhibits a viscosity of below about 1,000 centistokes, preferably below about 500 centistokes, more preferably below about 250 centistokes, and most preferably below 100 centistokes, at 25 °C.

The characterization of one embodiment of the polymer network as being swellable by the fluid means that the embodiment of the polymer network is capable of absorbing the fluid. In a preferred embodiment, the composition of polymer network is tailored to enhance its compatibility with the fluid. For example, if the polymer network is to be swollen with a hydrocarbon fluid, then the hydrocarbon character of the polymer network may be increased by increasing the number and/or the carbon chain length of the organic substituents of the polyfunctional organosilicone compound used to form the polymer network.

In a preferred embodiment, the fluid component of the present invention comprises an emollient compound. Suitable emollient compound include any fluid that provides emollient properties, that is, that when applied to skin, tend to remain on the surface of the skin or in the stratum corneum layer of the skin to act as lubricants, reduce flaking and to improve the appearance of the skin. Emollient compound are generically known and include, for example, hydrocarbons, such as for example, isododecane, isohexadecane, hydrogenated polyisobutene, organic waxes, such as for example, jojoba, silicone fluids, such as, for example, cyclopentasiloxane, dimethicone, bis-phenylpropyl dimethicone, esters, such as, for example, octyldodecyl neopentanoate, oleyl oleate, as well as fatty acids and alcohols, such as for example, oleyl alcohol, isomyristyl alcohol.

In a highly preferred embodiment, the fluid component of the present invention comprises a silicone fluid, more preferably a silicone fluid that exhibits emollient properties. Suitable silicone fluids include, for example, cyclic silicones of the formula D_{w}, wherein D is defined as above, R⁸ is preferably methyl, and r is an integer wherein 3 ≤ w ≤ 12, such as, for example, hexamethylcyclotrisiloxane ("D₃"), octamethylcyclotetrasiloxane ("D₄"), decamethylcyclopentasiloxane ("D₅"), and dodecamethylcyclohexasiloxane ("D₆") as well as linear or branched organopolysiloxanes having the formula (XVI):

M'D'ₓT'_{y}M' (XVI)

wherein:
M' is R²⁹₃SiO_{1/2};
D' is R³⁰₂SiO_{2/2}
T' is R³¹SiO_{3/2}
R²⁹, R³⁰ and R³¹ are each independently alkyl, aryl or aralkyl, and
x and yare each independently integers of from 0 to 300, preferably from 0 to 100, more preferably from 0 to 50 and most preferably from 0 to 20.

The polyfunctional organosilicone compound may, optionally, comprise any of several terminally unsaturated organic groups or alcohols such as allyl started polyethers, olefins, allyl esters, vinyl aromatics, cetyryl alcohol and the like. This may be done in a sequential manner with the polymerization occurring first followed by pendant addition, or by simultaneous reaction of all components if mixing occurs before catalyst addition.

In a preferred embodiment, the silicone material of the present invention is made by polymerizing the polyfunctional polymer in the presence of a silicone fluid and a polymerization catalyst.

In a preferred embodiment, the polymer network of the present invention is swollen with the fluid and is capable of serving as a reservoir or carrier for the fluid. The fluid is released from the silicone material by subjecting the silicone material to low shear, such as for example by rubbing the silicone material between one's fingers.

In a preferred embodiment, the polymer network of invention can be swollen by the fluid from a first volume to a swollen volume that is a factor of from 1.01 to 5000, more preferably from 2 to 1000, and even more preferably from 5 to 500, times the first volume. As used in the preceding sentence, the term "first volume" means the volume of the polymer network absent the fluid, such as, for example, in the case where the silicone material consists of the polymer network and a volatile fluid, the first volume corresponds to the volume of material remaining after evaporation of the volatile fluid from the silicone material.

The silicone material may be further processed under moderate to high shear to decrease the size of the particles to a desired level. This may be achieved, for example, by adding more silicone fluid, such as D₅ fluid, to the particulate material to thereby form a slurry and then subjecting the slurry to a moderate to high shearing force. The slurry may be subjected to high shear in, for example, a Sonolator apparatus, a Gaulin Homogenizer or a Micro Fluidizer apparatus.

In a preferred embodiment, the slurry is subjected to sufficient shearing to produce a silicone cream composition having comprising silicone material particles having an average particle size, as measured by light scattering, less than or equal to 600 µm, more preferably less than 400 µm, even more preferably less than 200 µm. A suitable technique for measuring particle size is disclosed in US Patent No. 5,929,162. In one preferred embodiment, the slurry is subjected to sufficient shearing to produce a silicone cream composition having comprising silicone material particles having an average particle size of less than 100 µm.

The personal care applications where the polymer network of the present invention may be employed include, but are not limited to, deodorants, antiperspirants, shaving products, skin lotions, moisturizers, toners, bath products, cleansing products, hair care products such as shampoos, conditioners, mousses, styling gels, hair sprays, hair dyes, hair color products, hair bleaches, waving products, hair straighteners, manicure products such as nail polish, nail polish remover, nails creams and lotions, cuticle softeners, protective creams such as sunscreen, inset repellent and anti-aging products, color cosmetics such as lipsticks, foundations, face powders, eye liners, eye shadows, blushes, makeup, mascaras and other cosmetic formulations where silicone components have been conventionally been added, as well as drug delivery systems for topical application of medicinal compositions that are to be applied to the skin.

In a preferred embodiment, the personal care composition of the present invention comprises one or more personal care ingredients. Suitable personal care ingredients include, for example, emollients, including, for example, the emollient fluids discussed above, moisturizers, humectants, water soluble dyes, liposoluble dyes, pigments, including pearlescent pigments such as, for example, bismuth oxychloride and titanium dioxide coated mica, colorants, fragrances, biocides, preservatives, antioxidants, antimicrobial agents, anti-fungal agents, antiperspirant agents, exfoliants, hormones, enzymes, medicinal compounds, vitamins, salts, electrolytes, alcohols, polyols, absorbing agents for ultraviolet radiation, botanical extracts, surfactants, silicone oils, organic oils, waxes, film formers, thickening agents such as, for example, fumed silica or hydrated silica, particulate fillers, such as for example, silica, talc, kaolin, starch, modified starch, mica, nylon, polyethylene powder, poly(methyl methacrylate) powder and clays, such as, for example, bentonite and organo-modified clays.

Suitable personal care compositions are made by combining, according to techniques known in the art, such as, for example, by mixing, one or more of the above components with compound the silicone composition of the present invention or with an emollient fluid and a polymerized product of a polyfunctional organosilicone. Suitable personal care compositions may be in the form of a single phase or in the form of an emulsion, including oil-in-water, water-in-oil and anhydrous emulsions, as well as multiple emulsions, such as, for example, oil-in water-in-oil emulsions and water-in-oil-in water-emulsions.

In a preferred embodiment, an antiperspirant composition comprises a silicone material according to the present invention and one or more active antiperspirant agents. Suitable antiperspirant agents include, for example, the Category I active antiperspirant ingredients listed in the U.S. Food and Drug Administration's October 10,1993 Monograph on antiperspirant drug products for over-the-counter human use, such as, for example, aluminum halides, aluminum hydroxyhalides, for example, aluminum chlorohydrate, and complexes or mixtures thereof with zirconyl oxyhalides and zirconyl hydroxyhalides, such as for example, aluminum-zirconium chlorohydrate, aluminum zirconium glycine complexes, such as, for example, aluminum zirconium tetrachlorohydrexgly.

In a preferred embodiment, a skin care composition comprises silicone material of the present invention and a vehicle, such as, for example, a silicone oil or an organic oil. The skin care composition may, optionally, further include emollients, such as, for example, triglyceride esters, wax esters, alkyl or alkenyl esters of fatty acids or polyhydric alcohol esters and one or more the known components conventionally used in skin care compositions, such as, for example, pigments, vitamins, such as, for example, Vitamin A, Vitamin C and Vitamin E, sunscreen or sunblock compounds, such as, for example, titanium dioxide, zinc oxide, oxybenzone, octylmethoxy cinnamate, butylmethoxy dibenzoylmethane, p-aminobenzoic acid and octyl dimethyl-p-aminobenzoic acid.

In a preferred embodiment, a color cosmetic composition, such as, for example, a lipstick, a makeup or a mascara composition comprises a silicone material according to the present invention, an emollient compound and one or more coloring agents, such as, for example, pigments, water soluble dyes or liposoluble dyes.

### Example 1

31 grams (0.00454 moles) of an organosilicone having the structural formula MD₅₀D^{H}₅₀M wherein M, D and D^{H} are each defined as-above, 3000 grams (.05211 moles) of divinylpolysiloxane of the structural formula M^{vi}D₉₀₀M^{vi}, wherein M^{vi} and D are defined as above and 100 ppm LPNC catalyst (added as 15.15 grams of a 2% solution of the catalyst in a 350 cSt polydimethylsiloxane fluid) were added together and heated to 90°C for two hours to make a polyfunctional organosilicone compound.

### Example 2

D^{vi}₄ (6.0 gram, 69.8 mmol of D^{vi}), MD^{H}₅₀M (14 grams, 221.4 mmol D^{H}), wherein D^{vi}, M and D^{H} are each defined as above, with R10 = ethenyl, and LPNC (2 mg) were stirred at 100°C. After 4 hours the percent solids (150°C, 45 min) had leveled off at 78 %, from an initial value of 67 %, indicative of equilibration.

### Example 3

D^{vi}₄ (13.8 gram, 160.4 mmol of D^{vi}), MD^{H}₅₀M (6.2 grams, 98.0 mmol D^{H}), wherein D^{vi}, M and D^{H} are each defined as above, with R10 = ethenyl, and LPNC (2 mg) were stirred at 100°C. After 4 hours the percent solids (150°C, 45 min) had leveled off at 62.5 %, from an initial value of 30 %, indicative of equilibration. Silicon-29 NMR analysis indicated random D^{H} (-37.5 ppm) and D^{vi} (-35.8 ppm) groups.

### Example 4

500 grams of the polyfunctional organosilicone compound of Example 1 was mixed with 1500 grams of D₅ silicone fluid and 0.2 grams of a 0.1 % solution of Karstadt's catalyst in a small dough mixer. The reaction mixture was then heated for 5 hours at 80°C to thereby polymerize the polyfunctional organosilicone compound to form a polymer network that entrapped the D₅ fluid. The reaction product was in the form of a powder.

The powdered product was then mixed with additional D₅ fluid to yield a slurry. The slurry was then subjected to high shear using a Gaulin Homogenizer at 8000 psi. The material was passed through the homogenizer four times to product a clear, high viscosity cream composition having a soft, silky feel.

### Example 5

500 grams (0.0093 moles) of polyfunctional organosilicone compound of Example 1was mixed with 1500 grams of D₅ silicone fluid and 19.5 grams (0.0093 moles) of a 2100 molecular weight allyl-started ethylene oxide/propylene oxide polyether in a dough mixer. 2 grams of Karstadt's catalyst was added and the mixture heated to 80°C for four hours to thereby polymerize the polyfunctional organosilicone compound and entrap the D₅ fluid. The reaction product was in the form of a white fluffy powder containing D₅.

The powdered reaction product was then mixed with additional D₅ fluid to yield a slurry. This was then subjected to high shear using a Gaulin Homogenizer at 8000 psi. The material was passed through the homogenizer four times to product a clear, high viscosity cream composition having a soft, silky feel.

### Example 6

500 grams (0.0093 moles) the polyfunctional organosilicone compound of Example 1 was mixed with 1500 grams of D₅ silicone fluid and 6.3 grams (0.0093 moles) of an allyl diester (isosteric acid, trimethylolpropane monoethylether ester) in a dough mixer. .2 grams of Karstadt's catalyst was added and the mixture heated to 80°C for four hours to thereby polymerize the polyfunctional organosilicone compound. The reaction product was in the form of a white fluffy powder containing D₅ fluid.

The powdered reaction product was then mixed with additional D₅ fluid to yield a slurry. The slurry was then subjected to high shear using a Gaulin Homogenizer at 8000 psi. The material was passed through the homogenizer four times to product a clear, high viscosity cream composition having a soft, silky feel.

### Example 7

500 grams (0.0093 moles) of the polyfunctional organosilicone compound of Example 1 was mixed with 1500 grams of D₅ silicone fluid and 2.5 grams (0.0093 moles) of α,β unsaturated C ₃₀₊ monoolefin in a dough mixer. .2 grams of Karstadt's catalyst was added and the mixture heated to 80°C for four hours. The material self polymerized as evidenced by the formation of a white fluffy powder containing D₅ fluid.

The powdered reaction product was then mixed with additional D₅ fluid to yield a slurry. The slurry was then subjected to high shear using a Gaulin Homogenizer at 8000 psi. The material was passed through the homogenizer four times to product a clear, high viscosity cream composition having a soft silky feel.

### Examples 8-11 and Comparative Example C1

The cream compositions of Examples 2 to 5 were used to make the antiperspirant composition of Examples 7-10 as set forth below in TABLE I.

The relative amounts for all ingredients of the compositions of the Examples 8-28 and Comparative Examples C1-C5 below are given in pbw per 100 pbw of the composition, with the notation "q.s." used with some ingredients, for example, a fragrance, where the amount of the ingredient is not critical, to indicate a non-measured sufficient amount of the ingredient.

**TABLE I**

| Component | Ex C1 | Ex 8 | Ex 9 | Ex 10 | Ex 11 |
|---|---|---|---|---|---|
| Silicone, Composition A | 60 | -- | -- | -- | -- |
| Silicone Composition of | -- | 60 | -- | -- | -- |
| Example 4 | | | | | |
| Silicone Composition of | -- | -- | 60 | -- | -- |
| Example 5 | | | | | |
| Silicone Composition of | -- | -- | -- | 60 | -- |
| Example 46 | | | | | |
| Silicone Composition of | -- | -- | -- | -- | 60 |
| Example 7 | | | | | |
| C₍₁₂₋₁₅₎alkyl benzoate | 10 | 10 | 10 | 10 | 10 |
| Talc | 3 | 3 | 3 | 3 | 3 |
| ZAG | 25 | 25 | 25 | 25 | 25 |
| Fragrance | q.s. | q.s. | q.s. | q.s. | q.s. |

The antiperspirant compositions of Examples 8-11 and Comparative Example C1 were evaluated for syneresis, tackiness, spreadability and sensory feel.

The compositions were evaluated for evidence of syneresis by maintaining the sample at room temperature, where syneresis would be indicated by separation onto two phases. Each of the compositions exhibited no evidence of syneresis after 1 week at room temperature.

The compositions were each evaluated for tackiness by applying a sample composition to a test subject's forearm, lightly placing the tester's fingertips on the area to which the composition was applied and then subjectively determining the degree of force required to separate the fingertips from the area, with tackiness indicated by a resistance imparted by the sample composition to separation of the fingertips from the area. None of the compositions exhibited a tacky feel.

The spreadability of the compositions was evaluated by spreading sample compositions on a test subject's forearm and evaluating the ease of spreading the sample composition over the skin and ranked as follows: 9>10>11>8 ~ C1.

The sensory feel of the compositions was evaluated by spreading sample compositions on a test subject's forearm and evaluating skin feel upon application or after drying. The composition of Comparative Example C1 exhibited a soft feel with a slight slipperiness. The composition of Example 9 exhibited a slight drag. The compositions of Examples 10 and 11 each exhibited a creamy, soft and smooth feel. The composition of Example 8 exhibited a dry feel.

### Example 12 and Comparative Example C2

The oil in water compositions of Example 12 and Comparative Example C2 were prepared by combining the ingredients set forth below in TABLE II as follows: Parts A and B were combined under homogenization and the ingredients of Part C were then added.

The compositions were evaluated for emulsion type, viscosity, stability, and sensory as follows. Emulsion type was identified by dilution technique. Both example C2 and 12 were oil in water emulsions. They were measured viscosity at 25°C after 24 hours using Brookfield viscometer. Results of these evaluations are set forth below in TABLE II. Sensory evaluation of emulsions was performed on skin by applying samples on forearms. Examples C2 and 12 provided easy rub in and Example 12 gave smooth, soft feel during rub in.

**TABLE II**

| | CEx C2 | Ex 12 |
|---|---|---|
| Components | | |
| Part A | | |
| Polypropylene glycol-2 Myristyl ether propionate | 5 | 5 |
| cyclopentasiloxane | 15 | -- |
| Silicone Composition of Example 4 | -- | 15 |

| Part B | | |
|---|---|---|
| Glycerin | 2 | 2 |
| Thickener (Carbopol 980, B.F. Goodrich) | 10 | 10 |
| Water | 64.9 | 64.9 |
| Polysorbate 20 | 2 | 2 |

| Part C | | |
|---|---|---|
| Triethanolamine | 0.1 | 0.1 |
| Preservative (Germaben II, International Specialty Products) | 1 | 1 |

| Properties | | |
|---|---|---|
| Emulsion type | oil-in-water | oil-in-water |
| Appearance after 24 hr at RT | milky lotion | milky lotion |
| Viscosity after 24 hr, cps | 2028 | 2808 |
| Appearance after 2 wk at RT | unchanged | unchanged |

### Examples 13-14 and Comparative Example C3

The oil in water compositions of Examples 13-14 and Comparative Example C3 were each prepared by combining the ingredients set forth below in TABLE III as follows: the ingredients of Part A were combined and mixed until uniform, the ingredients of Part B were mixed together and Part A was then added to Part B and mixed under homogenization. The triethanolamine was then slowly added to the mixture, followed by the preservative. When adding Part A and Part B together, C3 showed two phase separation immediately whereas example 13 and 14 developed a stable white emulsion.

Stability was measured by monitoring any appearance changes at room temperature after 24 hr and 2 weeks. Initial viscosity was measured by using Brookfield viscometer at 25°C after 24 hour. Results of these evaluations are set forth below in TABLE III. Sensory evaluation was performed by applying samples on human skin and compared skin feel to C3. Sample 13 and 14 gave soft smooth feel on the skin when compared to C3. Example 14 showed lower in spreadability and higher substantive skin feel compared to C3 and example 13. Examples 13 and 14 did not show tackiness.

**TABLE III**

| | CEx C3 | EX 13 | EX 14 |
|---|---|---|---|
| Components | | | |
| Part A | | | |
| Octyl dodecylneopentanoate | 5 | 5 | 5 |
| Cyclopentasiloxane | 30 | -- | -- |
| Silicone Composition of Example 4 | -- | 30 | -- |
| Silicone Composition of Example 5 | -- | -- | 30 |

| Part B | | | |
|---|---|---|---|
| Glycerin | 2 | 2 | 2 |
| Thickener (Carbopol 980, B.F. Goodrich) | 20 | 20 | 20 |
| Water | 41.9 | 41.9 | 41.9 |

| Part C | | | |
|---|---|---|---|
| Triethanolamine | 0.1 | 0.1 | 0.1 |

| Part D | | | |
|---|---|---|---|
| Preservative (Germaben II) | 1 | 1 | 1 |

| Properties | | | |
|---|---|---|---|
| Initial viscosity at 25 C (cps) | -- | 62088 | 56160 |
| Appearance after 24 hr at RT | separated | emulsion | emulsion |
| Appearance after 2 wk at RT | separated | very slight separation on the surface | unchanged |

### Examples 15-18 and Comparative Example C4

The water in oil emulsion compositions of Examples 15 to 18 and Comparative Example C4 were prepared by combining the ingredients set forth below in TABLE IV as follows: Part B was slowly added to Part A and mixed for 30 min. Such compositions are useful, for example, as lotions for skin and hair care applications.

Then evaluated sensory properties, measured viscosity and monitored stability at RT, 40°C and 3 cycles freeze/thaw (24hr/24hr). All exemplary silicone materials acted as thickening agents and as anti-syneresis agents in water-in-oil emulsions and extended product stability as compared to C4. Results of viscosity and stability evaluations are set forth below in TABLE IV. The compositions of Examples 15-18 each had a soft, cushioning, luxurious feel as compared tot hat of Comparative Example C4.

**TABLE IV**

| | Ex 15 | Ex 16 | Ex 17 | Ex 18 | CEx C4 |
|---|---|---|---|---|---|
| Components | | | | | |
| Part A | | | | | |
| Cyclopentasiloxane/ Dimethicone copolyol | 10 | 10 | 10 | 10 | 10 |
| Isododecane | 8 | 8 | 8 | 8 | 8 |
| Sorbitan oleate (Span 80, ICI) | 0.6 | 0.6 | 0.6 | 0.6 | 0.6 |
| Silicone Composition of Example 4 | 8 | -- | -- | -- | -- |
| Silicone Composition of Example 5 | -- | 8 | -- | | -- |
| Silicone Composition of Example 6 | -- | -- | 8 | -- | -- |
| Silicone Composition of Example 7 | -- | -- | -- | 8 | -- |
| Cyclopentasiloxane | -- | -- | -- | -- | 8 |

| Part B | | | | | |
|---|---|---|---|---|---|
| Butylene glycol | 2 | 2 | 2 | 2 | 2 |
| NaCl | 0.5 | 0.5 | 0.5 | 0.5 | 0.5 |
| Quaternium-15 | 0.1 | 0.1 | 0.1 | 0.1 | 0.1 |
| Water | 70.8 | 70.8 | 70.8 | 70.8 | 70.8 |

| Properties | | | | | |
|---|---|---|---|---|---|
| Appearance, 24 hr at RT | emulsion | emulsion | emulsion | emulsion | emulsion |
| Viscosity, 24 hr at RT | 16484 | 29016 | 17160 | 24960 | 3588 |
| Viscosity, 3 days at 40°C | 16484 | 26520 | 16692 | 23712 | 3588 |
| Viscosity, 1 wk at 40°C | 16484 | 24960 | 16224 | 22776 | 3588 |
| Viscosity, after 3 freeze/thaw cycles | 14040 | 29016 | 16848 | 24024 | 3120 |
| Appearance, 2 wk at RT | no change | no change | no change | no change | separation |

### Examples 19 -22 and Comparative Example C5

The anhydrous emulsion compositions of Examples 19-22 and Comparative Example C5 were prepared from the ingredients set forth below by adding Part B to Part A and mixing for 30 min after development of an emulsion.

The viscosity at 25°C and appearance of each composition was evaluated after 24 hour. Results of these evaluations are set forth below in TABLE V. Example 19-22 contained silicone compositions and they showed higher in viscosity and better product stability after 24 hours when compared to C5.

**TABLE V**

| | Ex 19 | Ex 20 | Ex 21 | Ex 22 | CEx C5 |
|---|---|---|---|---|---|
| Ingredients | | | | | |
| Part A | | | | | |
| Cyclopentasiloxane/ Dimethicone copolyol | 20 | 20 | 20 | 20 | 20 |
| Vitamin E | 0.25 | 0.25 | 0.25 | 0.25 | 0.25 |
| Silicone Composition of Ex 4 | 12.5 | -- | -- | -- | -- |
| Silicone Composition of Ex 5 | -- | 12.5 | -- | -- | -- |
| Silicone Composition of Ex 6 | -- | -- | 12.5 | -- | -- |
| Silicone Composition of Ex 7 | -- | -- | -- | 12.5 | -- |
| Cyclopentasiloxane | -- | -- | -- | -- | 12.5 |

| Part B | | | | | |
|---|---|---|---|---|---|
| Propylene glycol | 66.75 | 66.75 | 66.75 | 66.75 | 66.75 |
| NaCl | 0.5 | 0.5 | 0.5 | 0.5 | 0.5 |

| Properties | | | | | |
|---|---|---|---|---|---|
| Appearance, 24 hr at RT | emulsion | emulsion | emulsion | emulsion | separation |
| Viscosity (cps), after 24 hr at 25° C | 20904 | 22464 | 23712 | 27924 | 936 |

### Examples 23-28

An antiperspirant stick composition (Example 23) is made by blending antiperspirant active, the silicone material of Example 2 and the other ingredients listed below in TABLEVI at 60°C until uniform.

**TABLE VI**

| Ingredients | Relative Amount |
|---|---|
| Cyclopentasiloxane | 30 |
| Silicone Composition of Ex 4 | 13 |
| ZAG | 25 |
| Stearyl alcohol | 15 |
| Isopropyl myristate | 1 |
| Fragrance | 1 |
| Talc | 3 |
| Hydrogenated castor oil | 4 |
| Bis-phenylpropyldimethicone | 8 |

A vitamin C cream composition (Example 24) is prepared by combining the ingredients in the relative amounts listed below in TABLEVII.

**TABLE VII**

| Ingredients | Relative Amount |
|---|---|
| Caprylic/capric triglyceride | q.s. |
| Silicone Composition of Ex 4 | 82 |
| Vitamin C | 1 |

A lip product composition (Example 25) is prepared by blending 7 pbw pigment (D&C Red no.7, Ca lake), 3 pbw mica, 70 pbw of the silicone material of Ex 1 and 20 % pbw cyclopentasiloxane.

A pressed eye shadow composition (Example 26) is made by combining the ingredients in the relative amounts listed below in TABLE VIII by separately preparing Parts A and B and then mixing well Part A and B, blending dimethicone and the silicone material and adding to the batch and then pressing the composition so formed into suitable containers.

**TABLE VIII**

| Ingredients | Relative Amount |
|---|---|
| Part A | |
| Talc | 32 |
| Zinc stearate | 5 |
| Preservative (Imidazolidinyl Urea, (Germall 115, International Specialty Products)) | 0.15 |

| Part B | |
|---|---|
| MicA/Chromium oxide green/Titanium dioxide (Spectra-Pearl GNG) | 50 |

| Part C | |
|---|---|
| Dimethicone, 5 centistokes | q.s. |
| Silicone Composition of Ex 4 | 5 |

A hair conditioner composition (Example 27) is prepared by heating the ingredients listed in Table IX to 70°C and mixing well.

**TABLE IX**

| Ingredients | Relative Amount |
|---|---|
| Cetyl alcohol | 2 |
| Cetearyl alcohol | 2 |
| Glyceryl momostearate | 0.5 |
| Cetrimonium chloride | 1.5 |
| Water | q.s. |
| Silicone Composition of Ex 4 | 2 |

A shampoo composition (Example 28) is made by combining the ingredients in the relative amounts set forth below in TABLE X as follows: (1) mix together Part A, (2) add silicone emulsion to the batch and mix. The silicone emulsion is made by mixing 30 pbw the silicone material of Ex 4 with 2 pbw Brij 72, 2 pbw Brij 76 and 66 pbw deionized water.

**TABLE X**

| Ingredients | Relative Amount |
|---|---|
| Part A | |
| Sodium laureth sulfate | 56 |
| Cocamidoprpoyl betaine | 6.7 |
| Hydroxypropyltrimonium chloride (Jaguar C13S) | 0.2 |
| Thickener (Carbopol 980, B.F. Goodrich) | 20 |
| Triethanolamine | 0.1 |
| Water | q.s. |
| | |

| Part B | |
|---|---|
| Aqueous emulsion of Silicone Composition of Ex 2 | 4 |

Personal care compositions containing the polymerized product and an emollient fluid, whether the polymerized product and an emollient fluid are separately added or added in the form of the silicone material of the present invention, exhibit improved sensory feel and exhibit good stability, that is, a high resistance to phase separation.

## Claims

1. A silicone material, comprising:
a) a polymer network, said polymer network comprising a polymerized product of a polyfunctional organosilicone compound, said polyfunctional organosilicone compound comprising, on average per molecule of the compound, one or more alkenyl substituents and one or more silicon-bonded hydride substituents, and
(b) a fluid within the network.

2. The silicone material of claim 1, wherein the polyfunctional organosilicone compound comprises one or more first structural units of the formula R¹ₐSiO_{4-a/2}, wherein each R¹ is independently a monovalent hydrocarbon radical, provided that at least one R¹ group per unit is an alkenyl radical, and a is an integer wherein 0 ≤ a ≤ 3, and one or more second structural units of the formula R²_{b}SiO_{4-b/2}, wherein each R² is independently H or a monovalent hydrocarbon radical, provided that at least one R² group per unit is H, and b is an integer wherein 0 ≤ b ≤ 3.

3. The silicone material of claim 1, wherein the polyfunctional organosilicone compound comprises one or more organopolysiloxanes of the formula M_{c}M^{vi}_{d}M^{H}ₑD_{f}D^{vi}_{g}D^{H}ₕTᵢT^{vi}ⱼT^{H}ₖQₗ, wherein M is R³₃SiO_{1/2}, M^{vi} is R⁴₂R⁵SiO_{1/2}, M^{H} is R⁶₂R⁷SiO_{1/2} D is R⁸₂SiO_{2/2}, D^{vi} is R⁹R¹⁰SiO_{2/2}, D^{H} is R¹¹R¹²SiO_{2/2}, T is R¹³SiO_{3/2}, T^{vi} is R¹⁴SiO_{3/2}, T^{H} is R¹⁵SiO_{3/2}, Q is SiO_{4/2}, R³, R⁴, R⁶, R⁸, R⁹, R¹¹ and R¹³ are each monovalent non-alkenyl hydrocarbon radicals, R⁵, R¹⁰ and R¹⁴ are each alkenyl, R⁷, R¹² and R¹⁵ are each H, and c, d, e, f, g, h, i, j, k and 1 are each integers selected to provide polymer a having a viscosity of from 1 to 1,000,000 cSt and having a desired amount of alkenyl groups and silicon-bonded H radicals per molecule.

4. The silicone material of claim 3, wherein the polyfunctional organosilicone has the structural formula M^{Vi}DᵤD^{H}ᵥM^{Vi}, M is R³₃SiO_{1/2}, M^{vi} is R⁴₂R⁵SiO_{1/2}, D is R⁸₂SiO_{2/2}, D^{H} is R¹¹R¹²SiO_{2/2}, R⁴, R⁸ and R¹¹ are each independently monovalent non-alkenyl hydrocarbon radicals, R⁵ is alkenyl, and R¹² is H and u and v are each integers, wherein 0 ≤ u ≤ 1000, and 1 ≤ v < 10.

5. The silicone material of claim 1, wherein the fluid is selected from cyclic silicones of the formula D_{w}, wherein D is defined as above, R⁸ is preferably methyl, and r is an integer wherein 3 ≤ w ≤ 12, linear or branched organopolysiloxanes having the formula M'D'ₓT'_{y}M', wherein M' is R²⁹₃SiO_{1/2}; D' is R³⁰₂SiO_{2/2}, T' is R³¹SiO_{3/2}, R²⁹, R³⁰ and R³¹ are each independently alkyl, aryl or aralkyl, and x and y are each independently integers of from 0 to 300 and mixtures thereof.

6. A method for making a silicone material, comprising polymerizing a polyfunctional organosilicone compound, said polyfunctional organosilicone compound comprising, on average per molecule of the compound, one or more alkenyl substituents and one or more silicon-bonded hydride substituents, in the presence of a fluid to form a polymer network with the fluid within the network.

7. A method for making a polyfunctional organosiloxane compound, comprising equilibrating one or more linear or cyclic siloxanes with a silylhydride functional siloxane and an alkenyl functional siloxane in the presence of a linear phosphonitrile chloride equilibration catalyst.

8. A personal care composition comprising a polymerized product of a polyfunctional organosilicone compound, said polyfunctional organosilicone compound comprising, on average per molecule of the compound, one or more alkenyl substituents and one or more siliconbonded hydride substituents.

9. A method for making a personal care composition, comprising combining one or more personal care ingredients with a polymerized product of a polyfunctional organosilicone compound, said polyfunctional organosilicone compound comprising, on average per molecule of the compound, one or more alkenyl substituents and one or more silicon-bonded hydride substituents.

10. A method for reversibly imparting characteristics of a solid to a fluid, comprising combining the fluid with a polymer network, said network comprising a polymerized product of a polyfunctional organosilicone compound, said polyfunctional organosilicone compound comprising, on average per molecule of the compound, one or more alkenyl substituents and one or more silicon-bonded hydride substituents and said network being swellable by the fluid, so that the fluid is contained within the polymer network.
